# EUROPEAN PATENT SPECIFICATION

(11) **EP 3 607 940 B1**
(45) Date of publication and mention of the grant of the patent: **11.02.2026**
(21) Application number: 18917042.6
(22) Date of filing: 08.06.2018
(51) Int. Cl.: A61K 9/10, A61K 9/16, A61K 47/26, A61K 47/38, A61K 31/496, A61P 25/18

(54) **ARIPIPRAZOLE SUSTAINED-RELEASE MICROSPHERE AND PREPARATION METHOD THEREFOR**
MIKROKAPSELN MIT VERZÖGERTER FREISETZUNG VON ARIPIPRAZOL UND HERSTELLUNGSVERFAHREN DAFÜR
MICROSPHÈRE À LIBÉRATION PROLONGÉE D'ARIPIPRAZOLE ET SON PROCÉDÉ DE PRÉPARATION

(30) Priority: 16.05.2018 CN 201810468934
(43) Date of publication of application: 12.02.2020
(73) Proprietor: Livzon Pharmaceutical Group Inc., Zhuhai, Guangdong 519090 (CN)
(72) Inventor: CHEN, Bin, Zhuhai City Guangdong 519090 (CN); YIN, Xia, Zhuhai City Guangdong 519090 (CN); WANG, Yanqing, Zhuhai City Guangdong 519090 (CN); XU, Peng, Zhuhai City Guangdong 519090 (CN); YANG, Yuda, Zhuhai City Guangdong 519090 (CN); CHEN, Miaoli, Zhuhai City Guangdong 519090 (CN); YE, Weilun, Zhuhai City Guangdong 519090 (CN); LV, Linyan, Zhuhai City Guangdong 519090 (CN); XU, Huijuan, Zhuhai City Guangdong 519090 (CN); LU, Wenqi, Zhuhai City Guangdong 519090 (CN); KONG, Xiangsheng, Zhuhai City Guangdong 519090 (CN); JIANG, Xiaoman, Zhuhai City Guangdong 519090 (CN)
(74) Representative: Uexküll & Stolberg
(86) International application number: PCT/CN2018/090345
(87) International publication number: WO 2019/218409

(56) References cited:
- WO-A1-2017/059106
- WO-A2-2009/001697
- CN-A- 106 727 358
- CN-A- 106 727 358
- CN-A- 107 213 136
- US-A1- 2011 217 341

## Description

### Technical Field

The present invention relates to the field of pharmaceutical preparations, and in particular to an aripiprazole sustained release microsphere and a preparation method thereof.

### Background Art

Aripiprazole is a novel atypical anti-schizophrenia drug that has a two-way regulating effect on the nervous system, a stabilizer for the transmitter. Aripiprazole has very high affinities for D2, D3, 5-HT1A and 5-HT2A receptors, and produces an anti-schizophrenia effect by partial agonism of D2 and 5-HT1A receptors and antagonism of 5-HT2A receptor, and thereby is used in the treatment of various types of schizophrenia. The clinical trials abroad have shown that aripiprazole has significantly therapeutic effects on both positive and negative symptoms of schizophrenia, and can also improve the concomitant emotional symptoms and reduce the recurrence rate of schizophrenia. However, due to the particularity of patients suffering from mental diseases, the phenomenon of refusal to take medication is very prominent and long-term medication is required. Therefore, there is a need to develop dosage forms and long-acting preparations that can improve the compliance. Long-acting sustained release microsphere for injection has characteristics of reduced frequency of drug administration to a patient, convenient administration, good bioavailability, stable plasma concentration, no liver first-pass effect, high patient compliance, and the like. An aripiprazole long-acting sustained release microsphere therefore has a very high clinical value.

Currently, long-acting aripiprazole sustained release preparations are commercially available as Abilify Maintena and Arista, which are developed and produced by Otsuka (Japan) and ALkermes (USA), respectively. The two commercial available products now have the same defect, namely, oral administration of tablets is needed within 14 days after the administration to achieve the therapeutic effect due to a lower concentration of drug released at the early stage of administration, which fails to achieve an effective drug concentration. This administration mode still retains the inconvenience of a short-acting drug in clinical medication, and has a significant peak-valley effect during the early stage of administration, which brings about major side effects and overdose.

Patent application CN102133171A to ALKERMES Inc. discloses an aripiprazole microcrystalline preparation, rather than a microsphere product. Also, the patent application involves aripiprazole microcrystals having an average diameter of about 30 µm-80 µm. It is speculated according to the syringe needle corresponding to the particle size of the commercial available microsphere product that, it is required to use a 7- or 8-gauge needle (18-21G), which has a diameter of 0.5-0.8 mm, resulting significant pain in patients during injection. In addition, due to the large particle size of the drug, there is a need to increase the viscosity so that the drug particles in the suspension are always held in suspension, thereby causing the difficulty in injection of suspending agent. Furthermore, since the suspension is prone to precipitation before injection, there are needs to carry out aspiration of the suspended drug solution into the syringe slowly and continuously, and remain the state of suspension, otherwise the needle may be clogged. Complicated operations are also performed during injection administration, and an improper operation easily leads to the clog of needle and thus the failure of injection. The entire administration process needs to be completed by the medical personnel receiving special training, resulting in inapplicability of wide use in general medical institutions and inconvenience for the extension of medication.

Patent CN101742989B to Otsuka Inc. and the literature published by Shogo Hiraoka et al. (Preparation and Characterization of High-Content Aripiprazole-Loaded Core-Shell Structure Microsphere for Long-Release Injectable Formulation) relate to a microsphere with a core/shell structure, which also has the problems of inability to use a 5-gauge needle due to the excessively large microsphere particle size, the high requirements on injection operation, and the strong sense of pain in patients, etc. Meanwhile, the microspheres prepared according to the method disclosed in the patent produced a concentrated release of 10% of the drug on the first day, which may increase the risk of side effects due to the excessively high drug concentration. Most of the samples mentioned in the patent are the samples that can release for more than 2 months, but as to the major and severe side effects of schizophrenia drugs, the development of products released over one month is more advantageous in terms of safety and timeliness of risk control. The release mode of the developed core/shell structure is more dependent on the composition of core components, which is relatively single for the controlled release of drug, and thus a concentrated release may occur due to the rapid degradation of shell, or a discontinuous release phenomenon may occur because of the excessively slow release of the drug due to the slow degradation rate of shell.

Patent CN1870980B discloses a preparation method of sterile aripiprazole injection, and the preparation process of aripiprazole sterile injection involved in the patent requires grinding and other processes and a long preparation period. Meanwhile, the aseptic conditions required in the preparation process are easily destroyed. It is therefore not applicable to a large-scale industrial production.

The microspheres in patent applications CN105078898A and CN105310997A have an excessively low drug loading. The aripiprazole long-acting injections disclosed in patent CN102525915B, and patent applications CN103301461A and CN105012236A contain an oil for injection, which will lead to increased pain in patients during injection, thus are of less significance in clinical use.

The microsphere in patent application CN106727358A has a particle size which cannot be controlled within 20 µm, so that a 5-gauge needle cannot be used. Moreover, the release period of microsphere is 2 months.

CN107213136A generally discloses long-acting sustained release microspheres comprising aripiprazole and poly(lactic-co-glycolic acid) having a mean particle size of less than 200 µm and in particular microspheres comprising about 50% aripiprazole and about 50 wt.-% poly(lactic-co-glycolic acid) and having a molar ratio of lactide to glycolide of 70:30, an intrinsic viscosity of 0.47 dL/g, a weight average molecular weight of 40,000 and a particle diameter of 34-127 µm.

In the literature "D-Optimal Designing and Optimization of Long Acting Microsphere-Based Injectable Formulation of Aripiprazole" published by Tushar Nahata and Tulsi Ram Saini, disclosed is an aripiprazole microsphere having a drug loading within 30%, which cannot meet the requirements for clinically long-acting sustained release.

### Contents of the Invention

An object of the present invention is to provide an aripiprazole sustained release microsphere, which can quickly reach an effective drug concentration at the early stage of administration without the need for oral administration of aripiprazole tablets to achieve the therapeutic effect. Furthermore, the microsphere will not result in a burst release effect. In addition, since the microsphere of the present invention has an average particle size of less than 20 µm, it is applicable to a 5-gauge needle, thereby reducing the pain of patients during administration. Finally, the aripiprazole sustained release microsphere provided by the present invention has characteristics of not only a small particle size, but also a high drug loading, a high yield, high spheronization and an ability to adapt to a large-scale production.

Accordingly, an object of the present invention is to provide an aripiprazole sustained release microsphere.

Another object of the present invention is to provide a method for preparing the aripiprazole sustained release microsphere.

Yet another object of the present invention is to provide a suspension comprising the aripiprazole sustained release microspheres described above.

The technical solutions for achieving the objects of the present invention are as follows:
In one aspect, the present invention provides an aripiprazole sustained release microsphere comprising aripiprazole or a salt thereof, and a poly(lactide-co-glycolide) copolymer, wherein,
after being dissolved with solvent B, the microsphere exhibits a spherical network framework structure with reticular pores distributed in the sphere and aripiprazole or a salt thereof is filled in the pores, wherein solvent B is 10% acetic acid, 20% acetic acid or 10% ethyl acetate;
the microsphere has a volume average particle size D50 of less than 20 µm, the content of aripiprazole or a salt thereof is 65%-80% of the total weight of the microsphere, and the content of the poly(lactide-co-glycolide) copolymer is 20%-35% of the total weight of the microsphere, wherein the poly(lactide-co-glycolide) copolymer has an intrinsic viscosity of 0.2-0.55 dL/g, a weight average molecular weight of 15,000-60,000, and a molar ratio of lactide to glycolide of 50:50-75:25.

Solvent B is 10% acetic acid or 20% acetic acid or 10% ethyl acetate.

Preferably, the microsphere has a volume average particle size D50 of 10-13 µm.

Preferably, the content of aripiprazole or a salt thereof is 70%-75%, and more preferably 71% of the total weight of the microsphere.

Preferably, the poly(lactide-co-glycolide) copolymer has an intrinsic viscosity of 0.2-0.35 dL/g, and most preferably 0.2 dL/g.

Preferably, the content of the poly(lactide-co-glycolide) copolymer is 25%-30%, and most preferably 29% of the total weight of the microsphere.

Preferably, the poly(lactide-co-glycolide) copolymer has a weight average molecular weight of 20,000-40,000, further preferably 20,000-30,000, and most preferably 25,000.

Preferably, the poly(lactide-co-glycolide) copolymer has a molar ratio of lactide to glycolide of 50:50.

In another aspect, the present invention provides a method for preparing the aripiprazole sustained release microsphere, comprising the following steps:
1) aripiprazole or a salt thereof is mixed with a poly(lactide-co-glycolide) copolymer, added with dichloromethane, heated to a certain temperature, and shaken for dissolving, wherein the weight ratio of aripiprazole or a salt thereof to the poly(lactide-co-glycolide) copolymer is 5:2, and the temperature is 40-65°C; wherein the poly(lactide-co-glycolide) copolymer has an intrinsic viscosity of 0.2-0.55 dL/g, a weight average molecular weight of 15,000-60,000, and a molar ratio of lactide to glycolide of 50:50-75:25;
2) under the condition of controlled evaporation of dichloromethane, the solution obtained in step 1) is mixed with a PVA solution, controlled at a certain temperature after pH adjustment and stirred to obtain an emulsion, wherein the PVA solution has a concentration of 0.1%-1% (w/v), the pH is 9-14, the ratio of the volume (L) of the PVA solution to the weight (g) of aripiprazole or a salt thereof is 0.5-1.5:1, and the certain temperature is controlled at a temperature below 15°C in the first hour of step 2), after which the temperature is maintained or raised to 15°C-30°C for about 2 hours;
3) the emulsion obtained in the step 2) is cured, subjected to solvent evaporation over a period of time, solidified into spheres, and the spheres are collected by centrifugation, and lyophilized to obtain the microspheres having a volume average particle size D50 of less than 20 µm.

Preferably, in step 1), the ratio of the total weight of aripiprazole or a salt thereof and the poly(lactide-co-glycolide) copolymer to that of aripiprazole or a salt thereof, poly(lactide-co-glycolide) copolymer and dichloromethane is 9%-25% (w/w).

Preferably, in step 1), the weight ratio of dichloromethane to aripiprazole or a salt thereof is 4:1-10:1, and more preferably 8:1.

Preferably, in step 1), the poly(lactide-co-glycolide) copolymer has an intrinsic viscosity of 0.2-0.35 dL/g, and most preferably 0.2 dL/g.

Preferably, in step 1), the poly(lactide-co-glycolide) copolymer has a weight average molecular weight of 20,000-40,000, further preferably 20,000-30,000, and most preferably 25,000.

Preferably, in step 1), the poly(lactide-co-glycolide) copolymer has a molar ratio of lactide to glycolide of 50:50.

Preferably, in step 1), the temperature is 55°C; in some embodiments, when the temperature in the step is lower than 40°C, the mixture is difficult to dissolve; when the temperature in the step is too high, the control on experimental process is affected, and a potential safety hazard is easily caused.

Preferably, in step 1), the shaking is carried out under a condition of heating to 40-65°C.

Preferably, in step 2), the PVA solution has a concentration of 0.5%-1% (w/v), and most preferably 1% (w/v).

Preferably, in step 2), the ratio of the volume (L) of the PVA solution to the weight (g) of aripiprazole or a salt thereof is 1.24:1.

Preferably, in step 2), the volume ratio of dichloromethane to PVA added in step 1) is 1:40-1:250, and more preferably 1:205.

Preferably, in step 2), the pH is 10.

Preferably, in step 2), the certain temperature is controlled to be at 12°C in the first hour of step 2).

Preferably, in step 2), the stirring rate of stirring is 3,000 rpm.

Preferably, in step 3), the curing is carried out for 3 hours.

Preferably, in step 3), the microsphere has a volume average particle size D50 of 10-13 µm.

In still another aspect, the present invention provides a suspension comprising the aripiprazole sustained release microspheres of the present invention and a pharmaceutically acceptable carrier.

Preferably, the pharmaceutically acceptable carrier is selected from the group consisting of a suspending agent, a pH adjusting agent, an isoosmotic adjusting agent, a surfactant, water, and physiological saline; the suspending agent is selected from the group consisting of sodium carboxymethylcellulose, polyvinyl alcohol, polyvinylpyrrolidone, sodium alginate, and glycerin; the isoosmotic adjusting agent is selected from the group consisting of sodium chloride, glucose, mannitol, and sorbitol; and the surfactant is a nonionic surfactant selected from the group consisting of polysorbate series and poloxamer series.

In further embodiments, the aripiprazole sustained release microspheres of the present invention are prepared as a suspension (for injection) comprising the aripiprazole sustained release microspheres of the present invention, and a pharmaceutically acceptable carrier which comprises the following components by weight: 0-10% mannitol and 0-5% sodium carboxymethylcellulose; and the pH is adjusted to 6.5-7.5.

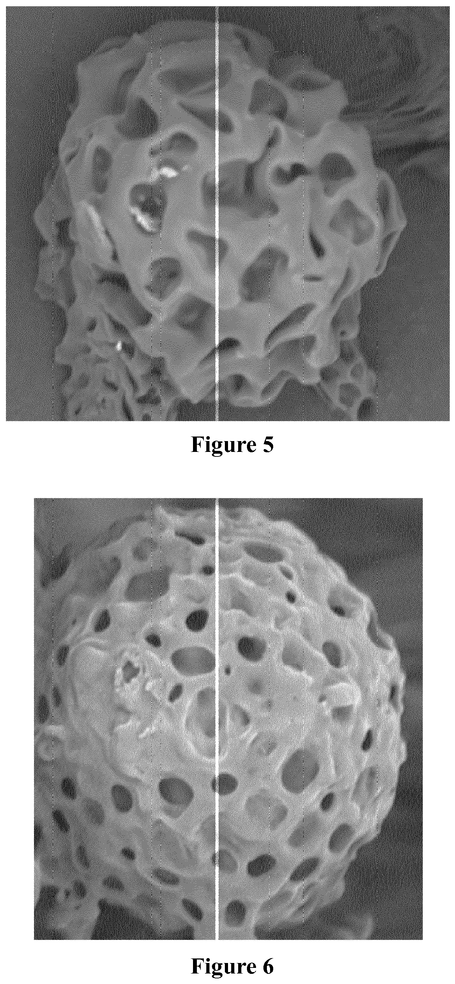

The aripiprazole sustained release microsphere of the present invention is dissolved in 20% acetic acid solution, sprayed with gold after natural air drying, and found by scanning electron microscopy observation that, the microspheres of the present invention have a spherical network framework structure, as shown in Figures 5 and 6.

Aripiprazole of the present invention includes aripiprazole crystals, non-crystal or amorphous forms, aripiprazole hydrates, or other polymorphs of aripiprazole.

The present invention also relates to the administration of the aripiprazole sustained release microsphere of the present invention to a subject in need thereof, thereby obtaining a sustained release profile in the patient, and the duration is at least 7 days, 14 days or 1 month.

The present invention has the following advantages:
1. The aripiprazole sustained release microspheres of the present invention release aripiprazole over at least one month after injection, and can reach an effective drug concentration within 14 days after administration without the need for oral administration. The release is stable at the later stage and can be completed within 30 days.
2. A burst release phenomenon may cause the drug concentration in human body to rise rapidly over a short period of time, and shorten the effective period of the drug, which is a key problem limiting the wide application of microspheres. Generally, the smaller the particle size of microsphere is, the greater the drug loading is, and the more severe the burst release phenomenon is. However, the aripiprazole sustained release microsphere of the present invention overcomes the above disadvantages, and the specific raw materials and the ratio thereof are selected such that the microsphere prepared according to the present invention has a good compatibility of active substance with the high-molecular polymer and a high porosity. The problem of burst release has been solved in the case that the average particle size of the microspheres is 10 µm and the drug loading is 68%-75%.
3. The existing microsphere products have a particle size of 20-100 µm, and can only be applicable to a 7- or 8-gauge needle (inner diameter: 0.51-0.84 mm), so that the patients have significant sense of pain during injection. The microspheres of the present invention have a narrower particle size distribution and a good uniformity, of which the average particle size is controlled within 20 µm, and thus can be injected with a 5-gauge needle. The compliance is consistent with those of conventional injections, thereby reducing the pain of patients. The needle gauges are shown in Table 1 below:

| International Gauge | Average Particle Size/µm | Needle Gauge | Needle Inner Diameter |
|---|---|---|---|
| 25G | 15 | 5 | 0.26 |
| 21G | 30 | 7 | 0.51 |
| 18G | 50 | 8 | 0.84 |

4. The suspensions used in the existing microsphere products require an increased viscosity to keep the drug particles always in suspension state, thereby resulting in difficulty in injection of the suspensions. Furthermore, since the suspension is prone to precipitation before injection, aspiration of the suspended drug solution into a syringe needs to be carried out slowly and continuously, and the suspended drug solution needs to be held in suspension state, otherwise the needle may be clogged. Complicated operations are also needed during injection administration, and an improper operation easily leads to the clog of needle and thus the failure of injection. The entire administration procedure needs to be completed by medical personnel receiving special training, resulting in inability to be widely used in general medical institutions and inconvenience for the extension of medication.

The experiments demonstrate that when the microspheres with an average particle size of less than 20 µm are dissolved using a suspending agent, the microsphere particles can be well dispersed and suspended in the solution for a long time without migration or aggregation. Aspiration and injection of the microsphere solution through a 5-gauge needle can be smoothly carried out with the well-dispersed suspending agent. If the average particle size of the microsphere particles is above 20 µm, sedimentation, migration, and aggregation may occur quickly, and the needle may be easily clogged.

Specifically, the microspheres of the present invention have an average particle size of less than 20 µm, and tend to suspend well in a suspension having a low viscosity and are not prone to precipitation. They are convenient for injection, which bring about convenience to the operation of medical staffs. Meanwhile, the use of a 5-gauge needle greatly reduces the sense of pain in patients.

Furthermore, the microspheres of the present invention having a particle size span within 1-2 µm have a stable quality, and tend to suspend during injection with no sedimentation, thus they are not prone to clogging the needle. The operation is simple without the need for additional training for medical staffs, and the medical staffs in the general medical institutions can operate them.

5. The aripiprazole sustained release microspheres of the present invention can achieve a drug loading of 65%-80% while maintaining a smaller particle size. Due to the use of a smaller amount of dichloromethane in the process and a high solid content, the yield is higher, reaching 3 g/L, while the existing microsphere process technology can only reach 1 g/L.

6. The aripiprazole sustained release microspheres of the present invention exhibit a spherical network framework structure, which is very advantageous for increasing the drug loading of the aripiprazole sustained release microsphere.

7. The present inventors have screened out the optimal ratio of aripiprazole to a poly(lactide-co-glycolide) copolymer through a large number of experiments. An excessively high or low weight ratio between them will both have a certain impact on the uniform release of the product. Here, the inventors have also found that in the step 2) of the preparation method of the present invention, the control of time and temperature is very important to the results of the experiments. In the first hour, if the temperature is too low, the API is easy to crystallize and solidify rapidly, resulting numerous microcrystals; and if the temperature is too high, more larger crystals are formed, destroying the microspheres, and thus forming irregular solid particles.

8. The preparation method of the aripiprazole sustained release microsphere provided by the present invention uses a simple process, with the stable and reproducible results, and can achieve an industrial large-scale production. Meanwhile, the resulting sustained release microspheres having a spherical network framework structure have a significantly smaller particle size and better pharmacodynamics and pharmacology than the existing microspheres having a core-shell structure.

### Description of the Drawings

Hereinafter, the embodiments of the present invention are described in detail in connection with the drawings, wherein:
Figure 1: a scanning electron microscope image of the microsphere sample prepared in Example 1;
Figure 2: a scanning electron microscope image of the microsphere sample prepared in Example 2;
Figure 3: a scanning electron microscope image of the microsphere sample prepared in Example 3;
Figure 4: a scanning electron microscope image of the microsphere sample prepared in Example 4;
Figure 5: a scanning electron microscope image of the microsphere sample prepared in Example 1 after being dissolved in 10% ethyl acetate;
Figure 6: a scanning electron microscope image of the microsphere sample prepared in Example 2 after being dissolved in 10% acetic acid;
Figure 7: a graph of particle size distribution of the microsphere sample prepared in Example 1;
Figure 8: a graph of plasma concentration-time curve of rats in Example 11;
Figure 9: a graph of plasma concentration-time curve of rats in Example 12;
Figure 10: a graph of plasma concentration-time curve of rats in Example 13;

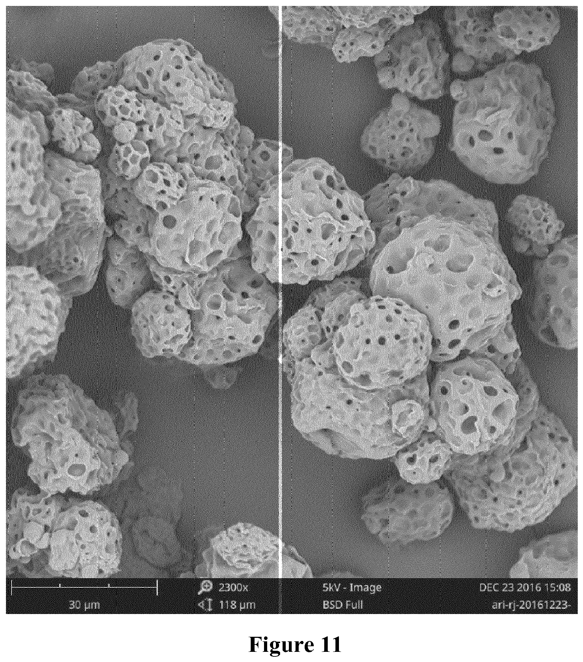

Figure 11: a scanning electron microscope image of the microsphere sample.

### Specific Modes for Carrying Out the Invention

The invention is described below with reference to the specific examples. Those skilled in the art can appreciate that these examples are only used to illustrate the present invention and do not limit the scope of the present invention in any way.

The experimental methods in the following examples are the conventional methods unless otherwise specified. The raw materials, reagent materials and the like used in the following examples are the commercially available products unless otherwise specified.

### Example 1

50g aripiprazole and 20g poly(lactide-co-glycolide) copolymer (0.2 dL/g) having a distribution coefficient of 1.5%, a weight average molecular weight of 25,000, and a molar ratio of lactide to glycolide of 50: 50 were mixed, added with 400g (301.9ml) dichloromethane and mixed. The mixture was heated to a temperature of 55°C and shaken for dissolving. At the same time, 1% PVA solution (62L) was formulated, and adjusted to pH 10 by adding sodium hydroxide. The temperature was controlled at 12°C, and the solvent was dispersed in the external PVA solution using a high speed emulsifier or a static mixer at a rotation speed of 3,000 rpm. After curing for 3 hours, the microspheres were collected by centrifugation and lyophilized.

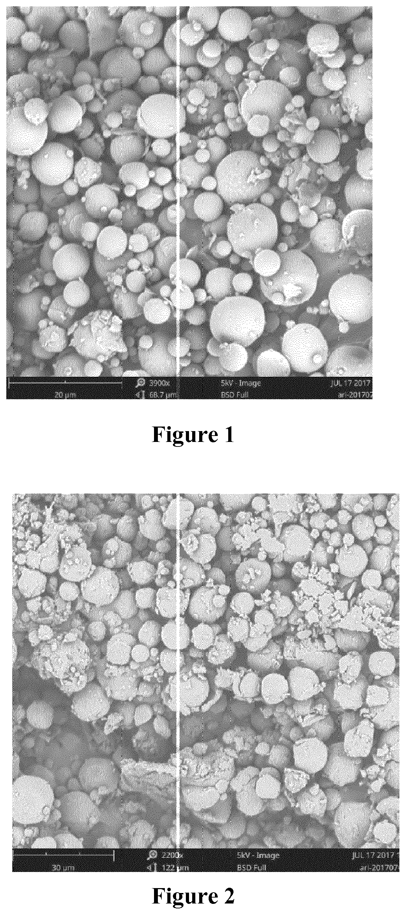

Conclusion: the microspheres have a drug loading of 71%, a yield of 92%, good spheronization, and smooth and complete microsphere surfaces. A scanning electron microscope image of the resulting microsphere sample is shown in Figure 1. When being dissolved in 10% ethyl acetate, the microspheres are found to have a spherical network framework structure (Figure 5). The plasma concentration shows a quick release at the early stage to ensure that an effective drug concentration is reached as soon as possible, and a stable release is completed at the later stage over one month.

### Example 2

50g aripiprazole and 20g poly(lactide-co-glycolide) copolymer (0.55 dL/g) having a distribution coefficient of 3.0%, a weight average molecular weight of 35,000, and a molar ratio of lactide to glycolide 50: 50 were mixed, added with 400g dichloromethane and mixed. The mixture was heated to a temperature of 55°C and shaken for dissolving. At the same time, 1% PVA solution (62L) was formulated, and adjusted to pH 10 by adding sodium hydroxide. The temperature was controlled at 12°C, and the solvent was dispersed in the external PVA solution using a high speed emulsifier or a static mixer at a rotation speed of 3,000 rpm. After curing for 3 hours, the microspheres were collected by centrifugation and lyophilized.

Conclusion: the microspheres have a drug loading of 70%, a yield of 87%, good spheronization, and smooth and complete microsphere surfaces. A scanning electron microscope image of the resulting microsphere sample is shown in Figure 2. When being dissolved in 10% acetic acid, the microspheres are found to have a spherical network framework structure (Figure 6). The plasma concentration shows a quick release at the early stage to ensure that an effective drug concentration is reached as soon as possible, and a stable release is completed at the later stage over one month.

### Example 3 (Reference)

50g aripiprazole and 20g poly(lactide-co-glycolide) copolymer (0.6 dL/g) having a distribution coefficient of 2.0%, a weight average molecular weight of 75,000, and a molar ratio of lactide to glycolide 75: 25 were mixed, added with 400g dichloromethane and mixed. The mixture was heated to a temperature of 55°C and shaken for dissolving. At the same time, 1% PVA solution (62L) was formulated, and adjusted to pH 10 by adding sodium hydroxide. The temperature was controlled at 12°C, and the solvent was dispersed in the external PVA solution using a high speed emulsifier or a static mixer at a rotation speed of 3,000 rpm. After curing for 3 hours, the microspheres were collected by centrifugation and lyophilized.

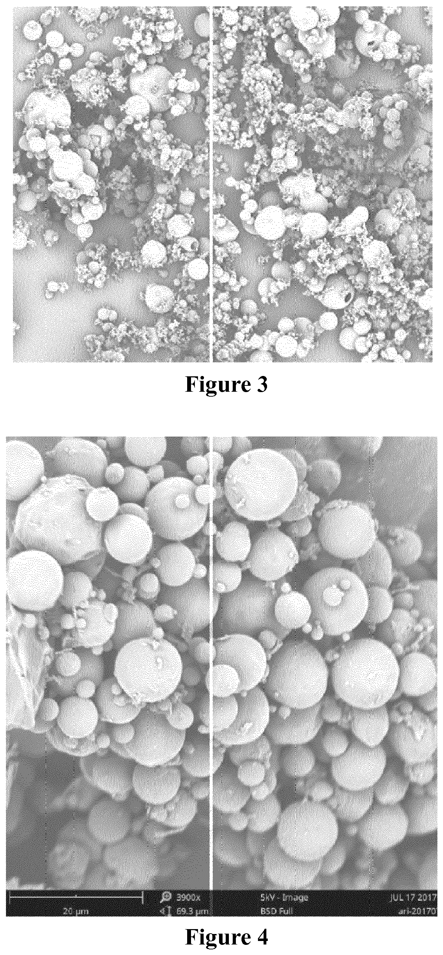

Conclusion: the microspheres have a drug loading of 68% and a yield of 80%. There are only a few spheres formed in the collected samples, and most of the samples are present as irregular particles and have a poor mobility. A scanning electron microscope image of the resulting microsphere sample is shown in Figure 3.

### Example 4

50g aripiprazole and 20g poly(lactide-co-glycolide) copolymer (0.35 dL/g) having a distribution coefficient of 1.0%, a weight average molecular weight of 40,000, and a molar ratio of lactide to glycolide 65: 35 were mixed, added with 400g dichloromethane and mixed. The mixture was heated to a temperature of 55°C and shaken for dissolving. At the same time, 1% PVA solution (62L) was formulated, and adjusted to pH 10 by adding sodium hydroxide. The temperature was controlled at 12°C, and the solvent was dispersed in the external PVA solution using a high speed emulsifier or a static mixer at a rotation speed of 3,000 rpm. After curing for 3 hours, the microspheres were collected by centrifugation and lyophilized.

Conclusion: the microspheres have a drug loading of 72%, a yield of 89.2%, good spheronization, and smooth and complete microsphere surfaces. A scanning electron microscope image of the resulting microsphere sample is shown in Figure 4.

### Example 5

50g aripiprazole and 20g poly(lactide-co-glycolide) copolymer (0.3 dL/g) having a distribution coefficient of 1.5%, a weight average molecular weight of 20,000, and a molar ratio of lactide to glycolide of 75: 25 were mixed, added with 400g dichloromethane and mixed. The mixture was heated to a temperature of 55°C and shaken for dissolving. At the same time, 1% PVA solution (62L) was formulated, and adjusted to pH 10 by adding sodium hydroxide. The temperature was controlled at 12°C, and the solvent was dispersed in the external PVA solution using a high speed emulsifier or a static mixer at a rotation speed of 3,000 rpm. After curing for 3 hours, the microspheres were collected by centrifugation and lyophilized.

Conclusion: the microspheres have a drug loading of 71.6%, a yield of 93.2%, good spheronization, and smooth and complete microsphere surfaces.

### Example 6 (Reference)

50g aripiprazole and 20g poly(lactide-co-glycolide) copolymer (0.2 dL/g) having a distribution coefficient of 1.5%, a weight average molecular weight of 40,000, and a molar ratio of lactide to glycolide 85: 15 were mixed, added with 400g dichloromethane and mixed. The mixture was heated to a temperature of 55°C and shaken for dissolving. At the same time, 1% PVA solution (62L) was formulated, and adjusted to pH 10 by adding sodium hydroxide. The temperature was controlled at 12°C, and the solvent was dispersed in the external PVA solution using a high speed emulsifier or a static mixer at a rotation speed of 3,000 rpm. After curing for 3 hours, the microspheres were collected by centrifugation and lyophilized.

Conclusion: the microspheres have a drug loading of 65.3%, a yield of 56%, and poor spheronization. A large amount of samples adhere together, with irregular particles and poor mobility.

### Example 7 (Reference)

50g aripiprazole and 20g poly(lactide-co-glycolide) copolymer (0.6 dL/g) having a distribution coefficient of 1.5%, a weight average molecular weight of 80,000, and a molar ratio of lactide to glycolide 100: 0 were mixed, added with 400g dichloromethane and mixed. The mixture was heated to a temperature of 55°C and shaken for dissolving. At the same time, 1% PVA solution (62L) was formulated, and adjusted to pH 10 by adding sodium hydroxide. The temperature was controlled at 12°C, and the solvent was dispersed in the external PVA solution using a high speed emulsifier or a static mixer at a rotation speed of 3,000 rpm. After curing for 3 hours, the microspheres were collected by centrifugation and lyophilized.

Conclusion: the microspheres have a drug loading of 53% and a yield of 61%. No microsphere is formed. There are irregular particles and a large number of fragments, with poor mobility.

### Example 8

50g aripiprazole (wherein aripiprazole accounted for 80% of the total weight of aripiprazole and poly(lactide-co-glycolide) copolymer) and 12g poly(lactide-co-glycolide) copolymer (0.2 dL/g) having a distribution coefficient of 1.5%, a weight average molecular weight of 30,000, and a molar ratio of lactide to glycolide 75: 25 were mixed, added with 200g dichloromethane (the content: 24% w/w) and mixed. The mixture was heated to a temperature of 65°C and shaken for dissolving. At the same time, 1% PVA solution (50L) was formulated, and adjusted to pH 12 by adding sodium hydroxide. The temperature was controlled at 12°C, and the solvent was dispersed in the external PVA solution using a high speed emulsifier or a static mixer at a rotation speed of 3,000 rpm. After curing for 3 hours, the microspheres were collected by centrifugation and lyophilized.

Conclusion: the microspheres have a drug loading of 73% and a yield of 91%, good spheronization, regular particles, round, complete and smooth surfaces, and good mobility.

### Example 9

50g aripiprazole (aripiprazole content: 80%) and 12g poly(lactide-co-glycolide) copolymer (0.3 dL/g) having a distribution coefficient of 2.0%, a weight average molecular weight of 60,000, and a molar ratio of lactide to glycolide 75: 25 were mixed, added with 250g dichloromethane (content: 20% w/w) and mixed. The mixture was heated to a temperature of 65°C and shaken for dissolving. At the same time, 1% PVA solution (62L) was formulated, and adjusted to pH 13 by adding sodium hydroxide. The temperature was controlled at 12°C, and the solvent was dispersed in the external PVA solution using a high speed emulsifier or a static mixer at a rotation speed of 3,000 rpm. After curing for 3 hours, the microspheres were collected by centrifugation and lyophilized.

Conclusion: the microspheres have a drug loading of 76% and a yield of 86%, good spheronization, regular particles, round, complete and smooth surfaces, and good mobility.

### Example 10

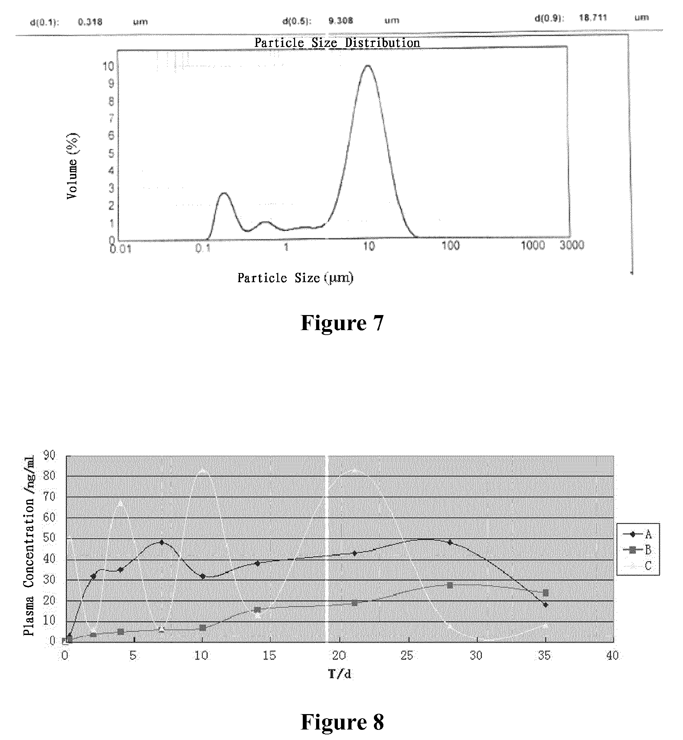

Aripiprazole sustained release microspheres were prepared and the particle sizes thereof were measured, as shown specifically in Table 2 below, wherein in Groups 1-9 the aripiprazole sustained release microspheres were those prepared in Examples 1-9 respectively; in Group 10 the aripiprazole microspheres were prepared as described in Example 9 of the patent CN201710052728, and in Group 11 the aripiprazole microspheres were prepared as described in Example 7 of the patent CN200880021585.8. Figure 7 is a graph showing the particle size distribution of the microsphere sample prepared in Example 1.

Measurement of samples: about 25 mg of the sample (approximately equivalent to 20 mg aripiprazole) was weighed, placed in a 10 ml vial, and added with 5 ml purified water by a pipette. The preparation was conducted in parallel in duplicate. The optical mode was set to Fraunhofer rfd, purified water was used as the dispersion medium, and the rotation speed was set to 2,200 rpm. The sample was suspended by sonication for 5 min before the measurement. The sample was firstly shaken up after being taken out, and slowly added dropwise into the sample cell with a dropper until the shading rate was 5%-10%, the sample adding was stopped and the results were determined and recorded (average value of 3 parallel measurements).

**Table 2: Measurement of particle size of aripiprazole sustained release microspheres**

| Experimental Group | Particle Size Range (D10/D90) | Average Particle Size (D50/µm) |
|---|---|---|
| 1 | 0.3/18.7 | 9.3 |
| 2 | 1.5/16.5 | 12.3 |
| 3 | 0.6/86.9 | 35.6 |
| 4 | 3.5/17.2 | 10.6 |
| 5 | 5.9/19.6 | 11.5 |
| 6 | 0.1/125.3 | 48.6 |
| 7 | 0.8/142.6 | 34.2 |
| 8 | 5.6/21.5 | 16.8 |
| 9 | 1.6/18.9 | 11.6 |
| 10 | 26.9/87.1 | 48.6 |
| 11 | 32.7/68.1 | 35.9 |

Conclusion: the average particle sizes of microspheres in the experimental groups 3, 6, 7, 10, 11 are greater than 20 µm, and the injection operation could not be performed using a 5-gauge needle.

### Example 11 Single dose I.M. long-acting study on SD rats

The aripiprazole long-acting microspheres prepared in Example 1 of the present invention were formulated into a preparation by the following method: the microspheres were weighed quantitatively, dispersed in a prepared suspension (the suspension comprising: 7% mannitol and 5% sodium carboxymethylcellulose; pH 6.8), so as to formulate a pharmaceutical preparation having a solid-liquid concentration ratio of 15% (w/v). A preparation of the microspheres prepared according to the method in Example 1 was used in Group A; a preparation, which was formulated using the microspheres prepared according to the method in Example 7 of the patent CN200880021585.8 and the same suspension, was used in Group B; and in group C the microsphere preparation in Group B was administered while aripiprazole tablets were administered orally (2 mg/kg/day).

The samples from the above three groups were injected into the thigh muscle of the rats at a dose of 25 mg/kg respectively, and for Group 3, the rats were orally administered at a dose of 2 mg/kg per day for the first 14 days after the injection administration. Blood samples were collected on days 1 (6 hours after injection), 2, 4, 7, 10, 14, 21, 28, and 35. The drug concentration in plasma was determined by an established LC-MS method. The plasma concentration-time curve of rats was plotted to evaluate the plasma concentration-time relationship.

The detailed results are shown in Figure 8.

It can be seen that the curve for Group A is relatively smooth, the plasma concentration is maintained in an effective concentration range during the administration, and the drug concentration is decreased after 30 days to ensure a complete release; the sustained release time in the curve for Group B is longer, which is more than one month, wherein the drug concentration increases slowly at the early stage, and there is a delay phenomenon; and in Group C the drug is administered orally, and there is a significant "peak-valley" phenomenon, and the plasma concentration fluctuates drastically.

### Example 12

The aripiprazole sustained release microspheres prepared in Examples 1, 2, 4, 5 and 8 of the present invention were formulated into preparations by the following method: the microspheres were weighed quantitatively, dispersed in a prepared suspension (the suspension comprising: 7% mannitol and 5% sodium carboxymethylcellulose; pH 6.8), and formulated into pharmaceutical preparations having a solid-liquid concentration ratio of 15% (w/v). For each group a microsphere preparation was prepared according to the method of the examples.

**Table 3: Corresponding relationship among various groups and the examples**

| Group | Corresponding Example |
|---|---|
| A | Example 1 |
| B | Example 2 |
| C | Example 4 |
| D | Example 5 |
| E | Example 8 |

The samples from the above three groups were injected into the thigh muscle of the rats at a dose of 25 mg/kg respectively, and for Group 3, an oral administration was conducted at a dose of 2 mg/kg per day for the first 14 days after the injection administration. Blood samples were collected on days 1 (6 hours after injection), 2, 4, 7, 10, 14, 21, 28, and 35. The drug concentration in plasma was measured by an established LC-MS method. The plasma concentration-time curve of rats was plotted to evaluate the plasma concentration-time relationship.

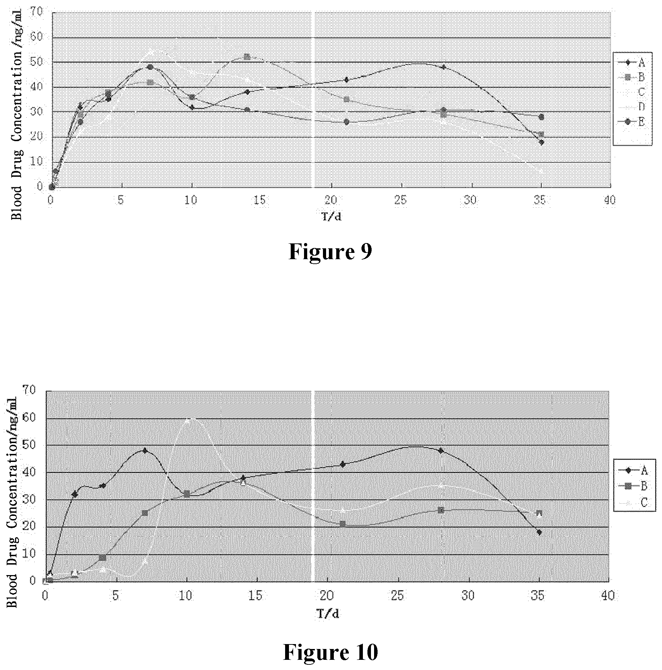

The detailed results are shown in Figure 9.

It can be seen that the curve for Group A is smooth, the plasma concentration is maintained in an effective concentration range during the administration, and the plasma concentration is decreased after 28 days to ensure a complete release; the curve for Group B is similar to that for Group A, the plasma concentration later decreases quickly after 20 days, but is within an effective concentration range; the plasma concentration in Group C decreases quickly after 20 days, which is similar to that in Group B, and both are within an effective concentration range; and the plasma concentrations in Groups D and E are relatively stable during the administration, and the drug concentration decreases rapidly in the later stage, but all are within an effective range.

### Example 13

The aripiprazole long-acting microspheres prepared in Example 1 of the present invention were prepared into a preparation by the following method: the microspheres were weighed quantitatively, dispersed in a prepared suspension (the suspension comprising: 7% mannitol and 5% sodium carboxymethylcellulose; pH 6.8), and formulated into a pharmaceutical preparation having a solid-liquid concentration ratio of 15% (w/v). A preparation of the microspheres prepared according to the method in Example 1 was used in Group A; a preparation, which was formulated with the microspheres prepared according to the method in Example 9 of the patent CN201710052728 and the same suspension, was used in Group B; and a preparation, which was formulated with the microspheres prepared according to the method in Example 1 of the patent CN201410219991 and the same suspension, was used in Group C.

The samples from the above three groups were injected into the thigh muscle of the rat at a dose of 25 mg/kg respectively, and for Group 3, an oral administration was conducted at a dose of 2 mg/kg per day for the first 14 days after the injection administration. Blood samples were collected on days 1 (6 hours after injection), 2, 4, 7, 10, 14, 21, 28, and 35. The drug concentration in plasma was measured by an established LC-MS method. The plasma concentration-time curve of rats was plotted to evaluate the plasma concentration-time relationship.

The results are shown in Figure 10.

It can be seen that the curve for Group A is smooth, the plasma concentration is maintained in an effective concentration range during the administration, and the plasma concentration decreases after 28 days to ensure a complete release; a delay phenomenon of plasma concentration occurs in Group B in the first 5 days, and the drug concentration increases slowly in the later stage to reach an effective concentration; and a delay phenomenon of plasma concentration occurs in group C in the first 7 days, and the plasma concentration increases rapidly in the later stage to reach an effective range.

### Example 14 Selection of PVA concentrations

50 g aripiprazole and 20 g poly(lactide-co-glycolide) copolymer (0.3 dL/g) having a distribution coefficient of 1.5%, a weight average molecular weight of 25,000, and a molar ratio of lactide to glycolide 50: 50 was mixed, and added with 400 g dichloromethane and mixed. The mixture was heated to a temperature of 55°C and shaken for dissolving.

A certain concentration of PVA solution (62 L) was formulated, and pH was adjusted to pH 10 by adding sodium hydroxide. The temperature was controlled at 12°C, and the solvent was dispersed in the external PVA solution using a high speed emulsifier or a static mixer at a rotation speed of 3000 rpm. After curing for 3 hours, the microspheres were collected by centrifugation and lyophilized.

**Table 4. Effects of different concentrations of PVA solutions on the experiment**

| Concentration of PVA Solution | Description of Experimental Results |
|---|---|
| 0.05% | Aggregation and adhesion occurred in the microsphere solution, with poor spheronization |
| 0.1% | Moderate spheronization |
| 0.5% | Relatively good spheronization |
| 1.0% | Good spheronization, the surfaces of the microspheres were smooth and complete |
| 1.5% | Moderate spheronization |

| | |
|---|---|
| Conclusion: when the concentration of PVA is 0.1%-1.0%, the experimental results are optimal, the obtained microspheres have good spheronization, and the surfaces are smooth and complete. | |

### Example 15: Effects of temperature on the experimental results

50 g aripiprazole and 20 g poly(lactide-co-glycolide) copolymer (0.3 dL/g) having a distribution coefficient of 1.5%, a weight average molecular weight of 25,000, and a molar ratio of lactide to glycolide of 50: 50 was mixed, and added with 400 g dichloromethane and mixed. The mixture was heated to a temperature of 55°C and shaken for dissolving. At the same time, 1% PVA solution (62 L) was formulated, and pH was adjusted to pH 10 by adding sodium hydroxide. The temperature was controlled at a certain temperature, and the solvent was dispersed in the external PVA solution using a high speed emulsifier or a static mixer at a rotation speed of 3,000 rpm. After curing for 3 hours, the microspheres were collected by centrifugation and lyophilized.

**Table 5. Effects of different temperatures on the experiment**

| Temperature (°C) | Description of Experimental Results |
|---|---|
| 5°C | Crystallization and solidification speeds were fast, and tiny crystals were formed |
| 10°C | Crystallization and solidification speeds were fast, and tiny crystals were formed |
| 12°C | Crystallization and solidification speeds were fast, and tiny crystals were formed |
| 15°C | Crystallization and solidification speeds were fast, and tiny crystals were formed |
| 20°C | Crystallization and solidification speeds were slow, and irregular solid particles appeared |

| | |
|---|---|
| Conclusion: when the temperature is lower than 15°C, the crystallization is rapid, tiny crystals are formed, the spheronization of microspheres is good, and the experimental results are optimal. | |

### Example 16 Effects of amount of dichloromethane on the experimental results

50 g aripiprazole and 20 g poly(lactide-co-glycolide) copolymer (0.3 dL/g) having a distribution coefficient of 1.5%, a weight average molecular weight of 25,000, and a molar ratio of lactide to glycolide of 50: 50 was mixed, and added with a certain amount of dichloromethane and mixed. The mixture was heated to a temperature of 55°C and shaken for dissolving.

1% PVA solution (62 L) was formulated and the temperature was controlled at 12°C. The pH was adjusted to pH 10 by adding sodium hydroxide. The solvent was dispersed in the external PVA solution using a high speed emulsifier or a static mixer at a rotation speed of 3,000 rpm. After curing for 3 hours, the microspheres were collected by centrifugation and lyophilized.

**Table 6. Effects of different amounts of dichloromethane solution on the experiment**

| Amount of Dichloromethane | Amount Ratio of Dichloromethane to Aripiprazole (weight ratio) | Description of Experimental Results |
|---|---|---|
| 150 g | 3:1 | Spheronization was poorer |
| 200 g | 4:1 | Drug loading was 72.8%, yield was 85%, and average particle size was 15.9 µm |
| 400 g | 8:1 | Drug loading of microspheres was 71%, yield was 92%, and average particle size was 9.3 µm |
| 500 g | 10:1 | Drug loading is 73.2%, yield was 89%, and average particle size is 7.5 µm |
| 600 g | 12:1 | Drug loading was 67.3%, yield was 75%, but spheronization was poor |
| 800 g | 14:1 | Drug loading was 42.0%, yield was 60%, and spheronization is poor |

| | | |
|---|---|---|
| Conclusion: it can be seen from the experimental data in Table 6 that when the amount ratio of dichloromethane to aripiprazole is 4:1-10:1, the experiment proceeds smoothly, and the resulting microsphere product is qualified; and when the amount ratio of dichloromethane to aripiprazole is 8:1, the experimental results are optimal, wherein Figure 11 is an electron microscope image of the microspheres obtained when the amount of dichloromethane is 800 g. | | |

### Example 17 Effects of amount of PVA solution on the experimental results

50 g aripiprazole and 20 g poly(lactide-co-glycolide) copolymer (0.3 dL/g) having a distribution coefficient of 1.5%, a weight average molecular weight of 25,000, and a molar ratio of lactide to glycolide 50: 50 was mixed, and added with 400 g dichloromethane and mixed. The mixture was heated to a temperature of 55°C and shaken for dissolving.

A certain amount of 1% PVA solution was formulated and the temperature was controlled at 12°C. The pH was adjusted to pH 10 by adding sodium hydroxide. The solvent was dispersed in the external PVA solution using a high speed emulsifier or a static mixer at a rotation speed of 3,000 rpm. After curing for 3 hours, the microspheres were collected by centrifugation and lyophilized.

**Table 7. Effects of different amounts of PVA solutions on the experiment**

| Amount of PVA Solution (L) | Amount Ratio of PVA to Aripiprazole (L:g) | Description of Experimental Results |
|---|---|---|
| 15 | 0.3:1 | Mostly microspheres were not formed, and fragments occurred |
| 25 | 0.5:1 | Spheronization was good, and surfaces were smooth |
| 50 | 1:1 | Spheronization was good, and surfaces were smooth |
| 60 | 1.2:1 | Spheronization was good, and surfaces were smooth |
| 75 | 1.5:1 | Spheronization was good, and surfaces were smooth |

| | | |
|---|---|---|
| Conclusion: it can be seen from the experimental results in Table 7 that when the amount ratio of PVA to aripiprazole is less than 0.5:1, the experimental results are very poor. | | |

### Example 18 Effects of pH adjustment on the experimental results

50 g aripiprazole and 20 g poly(lactide-co-glycolide) copolymer (0.3 dL/g) having a distribution coefficient of 1.5%, a weight average molecular weight of 25,000, and a molar ratio of lactide to glycolide 50: 50 was mixed, and added with 400 g dichloromethane and mixed. The mixture was heated to a temperature of 55°C and shaken for dissolving.

1% PVA solution (62 L) was formulated and the temperature was controlled at 12°C. The pH was adjusted by adding sodium hydroxide. The solvent was dispersed in the external PVA solution using a high speed emulsifier or a static mixer at a rotation speed of 3,000 rpm. After curing for 3 hours, the microspheres were collected by centrifugation and lyophilized.

**Table 8. Effects of different pH adjustments on the experiment**

| pH | Description of Experimental Results |
|---|---|
| 7 | Spheronization was not good |
| 8 | Spheronization was not good |
| 9 | Spheronization was good, and surfaces were smooth |
| 10 | Spheronization was good, and surfaces were smooth |
| 12 | Spheronization was good, and surfaces were smooth |

| | |
|---|---|
| Conclusion: when the pH is less than 9, the experimental results are poor. | |

## Claims

1. An aripiprazole sustained release microsphere comprising aripiprazole or a salt thereof and poly(lactide-co-glycolide), wherein
after being dissolved with solvent B, the microsphere exhibits a spherical network framework structure with reticular pores distributed in the sphere; and aripiprazole or a salt thereof is filled in the pores, wherein solvent B is 10% acetic acid, 20% acetic acid or 10% ethyl acetate;
the microsphere has a volume average particle size D50 of less than 20 µm; the content of aripiprazole or a salt thereof is 65%-80% of the total weight of the microsphere, and the content of the poly(lactide-co-glycolide) copolymer is 20%-35% of the total weight of the microsphere, wherein the poly(lactide-co-glycolide) copolymer has an intrinsic viscosity of 0.2-0.55 dL/g, a weight average molecular weight of 15,000-60,000, and a molar ratio of lactide to glycolide of 50:50-75:25.

2. The microsphere according to claim 1, wherein the microsphere has a volume average particle size D50 of 10-13 µm.

3. The microsphere according to claim 1 or 2, wherein the content of aripiprazole or a salt thereof is 70%-75%, and more preferably 71% of the total weight of the microsphere.

4. The microsphere according to any one of claims 1 to 3, wherein the poly(lactide-co-glycolide) copolymer has an intrinsic viscosity of 0.2-0.35 dL/g;
and/or
the poly(lactide-co-glycolide) copolymer has a weight average molecular weight of 20,000-40,000;
and/or
the poly(lactide-co-glycolide) copolymer has a molar ratio of lactide to glycolide of 50:50.

5. A method for preparing an aripiprazole sustained release microsphere, comprising the following steps:
1) aripiprazole or a salt thereof is mixed with a poly(lactide-co-glycolide) copolymer, added with dichloromethane, heated to a certain temperature, and shaken for dissolving, wherein the weight ratio of aripiprazole or a salt thereof to the poly(lactide-co-glycolide) copolymer is 5:2, and the temperature is 40-65°C; wherein the poly(lactide-co-glycolide) copolymer has an intrinsic viscosity of 0.2-0.55 dL/g, a weight average molecular weight of 15,000-60,000, and a molar ratio of lactide to glycolide of 50:50-75:25;
2) under the condition of controlled evaporation of dichloromethane, the solution obtained in step 1) is mixed with a PVA solution, and stirred at a certain controlled temperature after a pH adjustment to obtain an emulsion, wherein the PVA solution has a concentration of 0.1%-1% (w/v), the pH is 9-14, the ratio of the volume (L) of the PVA solution to the weight (g) of aripiprazole or a salt thereof is 0.5-1.5:1, and the certain temperature is controlled at a temperature below 15°C in the first hour of step 2), after which the temperature is maintained or raised to 15°C-30°C for about 2 hours;
3) the emulsion obtained in step 2) is cured, subjected to solvent evaporation over a period of time, solidified into spheres; and the spheres are collected by centrifugation, and lyophilized to obtain the microspheres having a volume average particle size D50 of less than 20 µm.

6. The method according to claim 5, wherein
in step 1), the ratio of the total weight of aripiprazole or a salt thereof and the poly(lactide-co-glycolide) copolymer to that of aripiprazole or a salt thereof, the poly(lactide-co-glycolide) copolymer and dichloromethane is 9%-25% (w/v);
and/or
in step 1), the weight ratio of dichloromethane to aripiprazole or a salt thereof is 4:1-10:1, and more preferably 8:1;
and/or
in step 1), the poly(lactide-co-glycolide) copolymer has an intrinsic viscosity of 0.2-0.35 dL/g;
and/or
in step 1), the poly(lactide-co-glycolide) copolymer has a weight average molecular weight of 20,000-40,000;
and/or
in step 1), the poly(lactide-co-glycolide) copolymer has a molar ratio of lactide to glycolide of 50:50;
and/or
in step 1), the temperature is 55°C;
and/or
in step 1), the shaking is carried out under a condition of heating to 40-65°C.

7. The method according to claim 5 or 6, wherein
in step 2), the PVA solution has a concentration of 0.5%-1% (w/v), and most preferably 1% (w/v);
and/or
in step 2), the ratio of the volume (L) of the PVA solution to the weight (g) of aripiprazole or a salt thereof is 1.24:1;
and/or
in step 2), the volume ratio of the added dichloromethane in step 1) to PVA is 1:40-1:250;
and/or
in step 2), the pH is 10;
and/or
the temperature is controlled to be at 12°C in the first hour of step 2);
and/or
in step 2), the stirring rate of stirring is 3,000 rpm.

8. The method according to any one of claims 5 to 7, wherein
in step 3), the curing is carried out for 3 hours;
and/or
in step 3), the microspheres have a volume average particle size D50 of 10-13 µm.

9. A suspension comprising the microsphere according to any one of claims 1-4 or the microsphere prepared by the method according to any one of claims 5-8, a pharmaceutically acceptable carrier and water for injection.

10. The suspension according to claim 9, wherein the pharmaceutically acceptable carrier is selected from the group consisting of a suspending agent, a pH adjusting agent, an isoosmotic adjusting agent, a surfactant, water, and physiological saline;
the suspending agent is selected from the group consisting of sodium carboxymethylcellulose, polyvinyl alcohol, polyvinylpyrrolidone, sodium alginate, or glycerin;
the isoosmotic adjusting agent is selected from the group consisting of sodium chloride, glucose, mannitol, or sorbitol;
the surfactant is a nonionic surfactant selected from the group consisting of polysorbate series and poloxamer series.

## Patentansprüche

1. Aripiprazol-Mikrosphäre mit verzögerter Freisetzung, die Aripiprazol oder ein Salz davon und Poly(lactid-co-glycolid) umfasst, wobei
die Mikrosphäre nach dem Auflösen mit Lösungsmittel B eine kugelförmige Netzwerkgerüststruktur mit in der Kugel verteilten retikulären Poren aufweist und die Poren mit Aripiprazol oder einem Salz davon gefüllt sind, wobei Lösungsmittel B 10%ige Essigsäure, 20%ige Essigsäure oder 10%iges Ethylacetat ist,
die Mikrosphäre eine volumengemittelte Partikelgröße D50 von weniger als 20 µm aufweist, der Gehalt an Aripiprazol oder einem Salz davon 65% bis 80% des Gesamtgewichts der Mikrosphäre beträgt und der Gehalt an dem Poly(lactid-co-glycolid)-Copolymer 20% bis 35% des Gesamtgewichts der Mikrosphäre beträgt, wobei das Poly(lactid-co-glycolid)-Copolymer eine intrinsische Viskosität von 0,2 bis 0,55 dL/g, ein gewichtsgemitteltes Molekulargewicht von 15.000 bis 60.000 und ein molares Verhältnis von Lactid zu Glycolid von 50:50 bis 75:25 aufweist.

2. Mikrosphäre nach Anspruch 1, bei der die Mikrosphäre eine volumengemittelte Partikelgröße D50 von 10 bis 13 µm aufweist.

3. Mikrosphäre nach Anspruch 1 oder 2, bei der der Gehalt an Aripiprazol oder einem Salz davon 70% bis 75% und vorzugsweise 71% des Gesamtgewichts der Mikrosphäre beträgt.

4. Mikrosphäre nach einem der Ansprüche 1 bis 3, bei der das Poly(lactid-co-glycolid)-Copolymer eine intrinsische Viskosität von 0,2 bis 0,35 dL/g aufweist und/oder
das Poly(lactid-co-glycolid)-Copolymer ein gewichtsgemitteltes Molekulargewicht von 20.000 bis 40.000 aufweist
und/oder
das Poly(lactid-co-glycolid)-Copolymer ein molares Verhältnis von Lactid zu Glycolid von 50:50 aufweist.

5. Verfahren zur Herstellung einer Aripiprazol-Mikrosphäre mit verzögerter Freisetzung, das die folgenden Schritte umfasst:
1) Aripiprazol oder ein Salz davon wird mit einem Poly(lactid-co-glycolid)-Copolymer gemischt, mit Dichlormethan versetzt, auf eine bestimmte Temperatur erhitzt und zum Auflösen geschüttelt, wobei das Gewichtsverhältnis von Aripiprazol oder einem Salz davon zu dem Poly(lactid-co-glycolid)-Copolymer 5:2 beträgt und die Temperatur 40 bis 65°C beträgt, wobei das Poly(lactid-co-glycolid)-Copolymer eine intrinsische Viskosität von 0,2 bis 0,55 dL/g, ein gewichtsgemitteltes Molekulargewicht von 15.000 bis 60.000 und ein molares Verhältnis von Lactid zu Glycolid von 50:50 bis 75:25 aufweist,
2) die in Schritt 1) erhaltene Lösung wird unter den Bedingungen einer kontrollierten Verdampfung von Dichlormethan mit einer PVA-Lösung gemischt und nach einer pH-Einstellung bei einer bestimmten geregelten Temperatur gerührt, um eine Emulsion zu erhalten, wobei die PVA-Lösung eine Konzentration von 0,1% bis 1% (w/v) aufweist, der pH-Wert 9-14 beträgt, das Verhältnis des Volumens (L) der PVA-Lösung zum Gewicht (g) von Aripiprazol oder einem Salz davon 0,5-1,5:1 beträgt und die bestimmte Temperatur in der ersten Stunde von Schritt 2) auf einer Temperatur unter 15°C geregelt wird, woraufhin die Temperatur für etwa 2 Stunden auf 15°C bis 30°C gehalten oder erhöht wird,
3) die in Schritt 2) erhaltene Emulsion wird ausgehärtet, über einen Zeitraum einer Lösungsmittelverdampfung unterworfen, zu Kugeln verfestigt und die Kugeln werden durch Zentrifugation gesammelt und lyophilisiert, um die Mikrosphären mit einer volumengemittelten Partikelgröße D50 von weniger als 20 µm zu erhalten.

6. Verfahren nach Anspruch 5, bei dem
in Schritt 1) das Verhältnis des Gesamtgewichts von Aripiprazol oder einem Salz davon und dem Poly(lactid-co-glycolid)-Copolymer zu dem von Aripiprazol oder einem Salz davon, dem Poly(lactid-co-glycolid)-Copolymer und Dichlormethan 9% bis 25% (w/v) beträgt
und/oder
in Schritt 1) das Gewichtsverhältnis von Dichlormethan zu Aripiprazol oder einem Salz davon 4:1-10:1 und vorzugsweise 8:1 beträgt
und/oder
in Schritt 1) das Poly(lactid-co-glycolid)-Copolymer eine intrinsische Viskosität von 0,2 bis 0,35 dL/g aufweist
und/oder
in Schritt 1) das Poly(lactid-co-glycolid)-Copolymer ein gewichtsgemitteltes Molekulargewicht von 20.000 bis 40.000 aufweist
und/oder
in Schritt 1) das Poly(lactid-co-glycolid)-Copolymer ein molares Verhältnis von Lactid zu Glycolid von 50:50 aufweist
und/oder
in Schritt 1) die Temperatur 55°C beträgt
und/oder
in Schritt 1) das Schütteln unter einer Bedingung des Erhitzens auf 40 bis 65°C durchgeführt wird.

7. Verfahren nach Anspruch 5 oder 6, bei dem
in Schritt 2) die PVA-Lösung eine Konzentration von 0,5% bis 1% (w/v) und am meisten bevorzugt 1% (w/v) aufweist
und/oder
in Schritt 2) das Verhältnis des Volumens (L) der PVA-Lösung zum Gewicht (g) von Aripiprazol oder einem Salz davon 1,24:1 beträgt
und/oder
in Schritt 2) das Volumenverhältnis des in Schritt 1) zugesetzten Dichlormethans zu PVA 1:40 bis 1:250 beträgt
und/oder
in Schritt 2) der pH-Wert 10 beträgt
und/oder
in der ersten Stunde von Schritt 2) die Temperatur auf 12°C geregelt wird und/oder
in Schritt 2) die Rührgeschwindigkeit 3.000 U/min beträgt.

8. Verfahren nach einem der Ansprüche 5 bis 7, bei dem
in Schritt 3) die Aushärtung 3 Stunden lang durchgeführt wird;
und/oder
in Schritt 3) die Mikrosphären eine volumengemittelte Partikelgröße D50 von 10 bis 13 µm aufweisen.

9. Suspension, die die Mikrosphäre gemäß einem der Ansprüche 1 bis 4 oder die nach dem Verfahren gemäß einem der Ansprüche 5 bis 8 hergestellte Mikrosphäre, einen pharmazeutisch annehmbaren Träger und Wasser für Injektionszwecke umfasst.

10. Suspension nach Anspruch 9, bei der der pharmazeutisch annehmbare Träger aus der Gruppe bestehend aus einem Suspendiermittel, einem pH-Regulator, einem isoosmotischen Regulator, einem oberflächenaktiven Mittel, Wasser und physiologischer Kochsalzlösung ausgewählt ist,
das Suspendiermittel aus der Gruppe bestehend aus Natriumcarboxymethylcellulose, Polyvinylalkohol, Polyvinylpyrrolidon, Natriumalginat oder Glycerin ausgewählt ist,
das isoosmotische Regulator aus der Gruppe bestehend aus Natriumchlorid, Glucose, Mannitol oder Sorbitol ausgewählt ist,
das oberflächenaktive Mittel ein nichtionisches oberflächenaktives Mittel ist, das aus der Gruppe bestehend aus der Polysorbat-Reihe und der Poloxamer-Reihe ausgewählt ist.

## Revendications

1. Microsphère à libération prolongée d'aripiprazole comprenant de l'aripiprazole ou un sel de celui-ci et du poly(lactide-co-glycolide), dans laquelle
après avoir été dissoute avec le solvant B, la microsphère présente une structure réticulaire sphérique à pores réticulaires répartis dans la sphère ; et l'aripiprazole ou un sel de celui-ci remplit les pores, le solvant B étant de l'acide acétique à 10 %, de l'acide acétique à 20 % ou de l'acétate d'éthyle à 10 % ;
la microsphère a une taille moyenne en volume de particule D50 inférieure à 20 µm ; la teneur en aripiprazole ou en un sel de celui-ci vaut 65 % - 80 % du poids total de la microsphère, et la teneur en le copolymère poly(lactide-co-glycolide) vaut 20 % - 35 % du poids total de la microsphère, le copolymère poly(lactide-co-glycolide) ayant une viscosité intrinsèque de 0,2 - 0,55 dl/g, une masse moléculaire moyenne en poids de 15 000 - 60 000, et un rapport molaire de lactide à glycolide de 50:50 - 75:25.

2. Microsphère selon la revendication 1, dans laquelle la microsphère a une taille moyenne en volume de particule D50 de 10 - 13 µm.

3. Microsphère selon la revendication 1 ou 2, dans laquelle la teneur en aripiprazole ou en un sel de celui-ci vaut 70 % - 75 %, et plus préférablement 71 % du poids total de la microsphère.

4. Microsphère selon l'une quelconque des revendications 1 à 3, dans laquelle le copolymère poly(lactide-co-glycolide) a une viscosité intrinsèque de 0,2 - 0,35 dl/g ;
et/ou
le copolymère poly(lactide-co-glycolide) a une masse moléculaire moyenne en poids de 20 000 - 40 000 ;
et/ou
le copolymère poly(lactide-co-glycolide) a un rapport molaire de lactide à glycolide de 50:50.

5. Procédé pour la préparation d'une microsphère à libération prolongée d'aripiprazole, comprenant les étapes suivantes :
1) l'aripiprazole ou un sel de celui-ci est mélangé avec un copolymère poly(lactide-co-glycolide), additionné de dichlorométhane, chauffé jusqu'à une certaine température et agité pour la dissolution, le rapport en poids d'aripiprazole ou d'un sel de celui-ci au copolymère poly(lactide-co-glycolide) étant de 5:2, et la température étant de 40-65 °C ; le copolymère poly(lactide-co-glycolide) ayant une viscosité intrinsèque de 0,2 - 0,55 dl/g, une masse moléculaire moyenne en poids de 15 000 - 60 000, et un rapport molaire de lactide à glycolide de 50:50 - 75:25 ;
2) dans la condition d'évaporation contrôlée du dichlorométhane, la solution obtenue dans l'étape 1) est mélangée avec une solution de PVA, puis agitée à une certaine température régulée après un ajustement du pH pour l'obtention d'une émulsion, la solution de PVA ayant une concentration de 0,1 % - 1 % (p/v), le pH étant de 9-14, le rapport du volume (L) de la solution de PVA au poids (g) d'aripiprazole ou d'un sel de celui-ci valant 0,5-1,5:1, et la certaine température étant régulée à une température inférieure à 15 °C pendant la première heure de l'étape 2), à la suite de quoi la température est maintenue ou élevée à 15°C - 30 °C pendant environ 2 heures ;
3) l'émulsion obtenue dans l'étape 2) est durcie, soumise à une évaporation du solvant pendant un espace de temps, solidifiée en sphères ; et les sphères sont recueillies par centrifugation, puis lyophilisées pour l'obtention des microsphères ayant une taille moyenne en volume de particule D50 inférieure à 20 µm.

6. Procédé selon la revendication 5, dans lequel
dans l'étape 1), le rapport du poids total d'aripiprazole ou d'un sel de celui-ci et du copolymère poly(lactide-co-glycolide) à celui d'aripiprazole ou d'un sel de celui-ci, du copolymère poly(lactide-co-glycolide) et de dichlorométhane vaut 9 % - 25 % (p/v) ;
et/ou
dans l'étape 1), le rapport en poids de dichlorométhane à aripiprazole ou un sel de celui-ci vaut 4:1 - 10:1, et plus préférablement 8:1 ;
et/ou
dans l'étape 1), le copolymère poly(lactide-co-glycolide) a une viscosité intrinsèque de 0,2 - 0,35 dl/g ;
et/ou
dans l'étape 1), le copolymère poly(lactide-co-glycolide) a une masse moléculaire moyenne en poids de 20 000 - 40 000 ;
et/ou
dans l'étape 1), le copolymère poly(lactide-co-glycolide) a un rapport molaire de lactide à glycolide de 50:50 ;
et/ou
dans l'étape 1), la température est de 55 °C ;
et/ou
dans l'étape 1), l'agitation est effectuée dans une condition de chauffage à 40 - 65 °C.

7. Procédé selon la revendication 5 ou 6, dans lequel
dans l'étape 2), la solution de PVA a une concentration de 0,5 % - 1 % (p/v), et le plus préférablement de 1 % (p/v) ;
et/ou
dans l'étape 2), le rapport du volume (L) de la solution de PVA au poids (g) d'aripiprazole ou d'un sel de celui-ci est 1,24:1 ;
et/ou
dans l'étape 2), le rapport en volume du dichlorométhane ajouté dans l'étape 1) au PVA vaut 1:40 - 1:250 ;
et/ou
dans l'étape 2), le pH est égal à 10 ;
et/ou
la température est régulée pour être à 12 °C pendant la première heure de l'étape 2) ;
et/ou
dans l'étape 2), la vitesse d'agitation de l'agitation est de 3,000 tours/min.

8. Procédé selon l'une quelconque des revendications 5 à 7, dans lequel
dans l'étape 3), le durcissement est effectué pendant 3 heures ;
et/ou
dans l'étape 3), les microsphères ont une taille moyenne en volume de particule D50 de 10 - 13 µm.

9. Suspension comprenant la microsphère selon l'une quelconque des revendications 1-4 ou la microsphère préparée par le procédé selon l'une quelconque des revendications 5-8, un véhicule pharmaceutiquement acceptable et de l'eau pour injection.

10. Suspension selon la revendication 9, dans laquelle le véhicule pharmaceutiquement acceptable est choisi dans l'ensemble constitué par un agent de suspension, un agent d'ajustement du pH, un agent d'ajustement iso-osmotique, un tensioactif, de l'eau et une solution saline physiologique ;
l'agent de suspension est choisi dans l'ensemble constitué par la carboxyméthylcellulose sodique, le poly(alcool vinylique), la polyvinylpyrrolidone, l'alginate sodique, et le glycérol ;
l'agent d'ajustement iso-osmotique est choisi dans l'ensemble constitué par le chlorure de sodium, le glucose, le mannitol, et le sorbitol ;
le tensioactif est un tensioactif non ionique choisi dans l'ensemble constitué par la série des polysorbates et la série des poloxamères.
